# EUROPÄISCHE PATENTSCHRIFT

(11) **EP 3 713 492 B1**
(45) Veröffentlichungstag und Bekanntmachung des Hinweises auf die Patenterteilung: **18.02.2026**
(21) Anmeldenummer: 18811761.8
(22) Anmeldetag: 23.11.2018
(51) Int. Cl.: A61B 6/03, A61B 6/00, G01R 33/42, G01R 33/48, G01T 1/29, A61B 5/00, A61B 6/10, A61B 6/42, A61B 6/50, A61B 5/055, G01R 33/422, G01T 1/16

(54) **PET-DETEKTOR FÜR EINEN KOMBINIERTEN PET/MRI-SCANNER**
PET-DETECTOR FOR A COMBINED PET/MRI-SCANNER
DÉTECTEUR DE TOMOGRAPHIE PAR ÉMISSION DE POSITRONS (TEP) POUR UN SCANNER TEP-IRM COMBINÉ

(30) Priorität: 24.11.2017 DE 102017221038
(43) Veröffentlichungstag der Anmeldung: 30.09.2020
(73) Patentinhaber: RWTH Aachen, 52062 Aachen (DE)
(72) Erfinder: GEBHARDT, Pierre, 53121 Bonn (DE); SCHUG, David, 52080 Aachen (DE); DEY, Thomas, 52064 Aachen (DE); GROSS-WEEGE, Nicolas, 52070 Aachen (DE); SCHULZ, Volkmar, 52146 Würselen (DE); WEISSLER, Bjoern, 52064 Aachen (DE)
(74) Vertreter: RCD-Patent Giesen, Schmelcher & Griebel Patentanwälte PartG mbB
(86) Internationale Anmeldenummer: PCT/EP2018/082344
(87) Internationale Veröffentlichungsnummer: WO 2019/101909

(56) Entgegenhaltungen:
- US-A1- 2012 330 131
- US-A1- 2016 103 194
- US-A1- 2017 164 915
- US-A1- 2017 299 675
- BO J PENG ET AL: "Studies of the interactions of an MRI system with the shielding in a combined PET/MRI scanner", PHYSICS IN MEDICINE AND BIOLOGY, vol. 55, no. 1, 11 December 2009 (2009-12-11), Bristol GB, pages 265 - 280, XP055548330, ISSN: 0031-9155, DOI: 10.1088/0031-9155/55/1/016
- ARNE BERNEKING ET AL: "Design and Characterization of a Gradient-Transparent RF Copper Shield for PET Detector Modules in Hybrid MR-PET Imaging", IEEE TRANSACTIONS ON NUCLEAR SCIENCE., vol. 64, no. 5, 1 May 2017 (2017-05-01), US, pages 1118 - 1127, XP055548334, ISSN: 0018-9499, DOI: 10.1109/TNS.2017.2691546
- J D THIESSEN ET AL: "MR-compatibility of a high-resolution small animal PET insert operating inside a 7 T MRI", PHYSICS IN MEDICINE AND BIOLOGY, vol. 61, no. 22, 21 November 2016 (2016-11-21), Bristol GB, pages 7934 - 7956, XP055548327, ISSN: 0031-9155, DOI: 10.1088/0031-9155/61/22/7934

## Beschreibung

Die Erfindung betrifft einen PET-Detektor für einen kombinierten PET/NRI-Scanner.

### Hintergrund der Erfindung

Die Positronen-Emissions-Tomographie (PET) ist ein bildgebendes Verfahren der Nuklearmedizin und stellt eine Variante der Emissionscomputertomographie dar. Mittels PET können Schnittbilder von lebenden Organismen erzeugt werden, indem die Verteilung einer radioaktiven Substanz, sogenannter Tracer, sichtbar gemacht wird. Hierdurch lassen sich biochemische und physiologische Funktionen abbilden.

PET beruht auf der gleichzeitigen Detektion zweier Gammastrahlungsphotonen, die nach dem Zerfall eines Positronen emittierenden Radionuklids entstehen (β+-Zerfall). Bei der Wechselwirkung eines Positrons mit einem Elektron (Annihilation) im Körper werden zwei hochenergetische Photonen in entgegengesetzte Richtungen ausgesandt. Diese Strahlung wird auch als Vernichtungsstrahlung bezeichnet.

Das PET-Gerät enthält typischerweise viele ringförmig um den Patienten angeordnete Detektoren für die Photonen. Das Prinzip der PET-Untersuchung besteht darin, Koinzidenzen zwischen je zwei gegenüberliegenden Detektoren aufzuzeichnen. Aus der zeitlichen und räumlichen Verteilung dieser registrierten Zerfallsereignisse wird auf die räumliche Verteilung der radioaktiven Substanz im Körperinneren geschlossen und eine Serie von Schnittbildern errechnet.

Da die Absorption der Photonen nur von der Dicke des durchstrahlten Gewebes, nicht jedoch vom Entstehungsort der Photonen abhängt, ermöglicht dies zudem eine genaue Quantifizierung der Verteilung der radioaktiven Substanz im Untersuchungsvolumen.

Ein Großteil der bisherigen PET Scanner basiert darauf, dass die zwei hochenergetischen Photonen (gamma photonen) in Kristallen gestoppt werden, in denen ein Szintillationsprozess optische Photonen erzeugt. Die Kristalle werden deshalb häufig auch als Szintillationskristalle bezeichnet. Die Photonen werden anschließend durch optische Sensoren aufgenommen und in elektrische Impulse gewandelt. Typischerweise werden als optische Sensoren Photomultiplier verwendet. Diese sind jedoch großvolumig und führen zu nicht unerheblichen Einschränkungen in Bezug auf die Größe. Wird eine hohe Auflösung benötigt, so muss eine große Zahl Photomultiplier angeordnet werden, die zu großen Apparaten führt.

Die Magnetresonanztomographie, abgekürzt MRT ist ein bildgebendes Verfahren, das vor allem in der medizinischen Diagnostik zur Darstellung von Struktur und Funktion der Gewebe und Organe im Körper eingesetzt wird. Es basiert physikalisch auf den Prinzipien der Kernspinresonanz, insbesondere der Feldgradienten- Kernspinresonanz, und wird daher auch als Kernspintomographie bezeichnet. Die ebenfalls zu findende Abkürzung MRI stammt von der englischen Bezeichnung Magnetic Resonance Imaging und wird im Nachfolgenden gleichbedeutend verwendet werden.

Mit der MRI kann man Schnittbilder des menschlichen (oder tierischen) Körpers erzeugen, die eine Beurteilung der Organe und vieler krankhafter Organveränderungen erlauben. Sie basiert auf - in einem Magnetresonanztomographiesystem erzeugten - sehr starken Magnetfeldern sowie magnetischen Wechselfeldern im Hochfrequenzbereich, mit denen bestimmte Atomkerne im Körper resonant angeregt werden, wodurch in einem Empfängerstromkreis ein elektrisches Signal induziert wird. Eine wesentliche Grundlage für den Bildkontrast sind unterschiedliche Relaxationszeiten verschiedener Gewebearten. Daneben trägt auch der unterschiedliche Gehalt an Wasserstoff-Atomen in verschiedenen Geweben (z. B. Muskel, Knochen) zum Bildkontrast bei.

Das Verfahren beruht darauf, dass die Atomkerne im untersuchten Gewebe durch eine Kombination von statischen und hochfrequenten magnetischen Feldern gezielt phasensynchron zu einer bestimmten Bewegung angeregt werden und dann ein messbares Signal in Form einer Wechselspannung abgeben, bis die Bewegung abgeklungen ist. Diese Bewegung heißt Larmorpräzession und ist mechanisch analog an einem Spielzeugkreisel zu beobachten, wenn seine Drehachse nicht senkrecht steht, sondern um die Senkrechte herum eine Präzession vollführt. Sowohl zur Anregung als auch zur Beobachtung des Signals ist eine Resonanzbedingung zu erfüllen, mit deren Hilfe es mittels inhomogener statischer Magnetfelder möglich ist, den Ort der präzedierenden Kerne zu ermitteln.

Einige Atomkerne (wie etwa die Wasserstoffkerne) in den Molekülen des zu untersuchenden Gewebes besitzen einen Eigendrehimpuls (Kernspin) und sind dadurch magnetisch. Diese Kerne erzeugen nach dem Anlegen eines starken statischen Magnetfeldes eine kleine longitudinale Magnetisierung in Richtung des statischen Feldes (Paramagnetismus). Durch ein kurzzeitig angelegtes zusätzliches hochfrequentes Wechselfeld im Radiofrequenzbereich lässt sich diese Magnetisierung aus der Richtung des statischen Feldes auslenken (kippen), also teilweise oder ganz (Sättigung) in eine transversale Magnetisierung umwandeln. Die transversale Magnetisierung beginnt sofort um die Feldrichtung des statischen Magnetfeldes zu präzedieren, d. h. die Magnetisierungsrichtung rotiert (siehe Abbildung zur Präzession). Diese Präzessionsbewegung der Gewebemagnetisierung induziert wie die Rotation des Magneten im Dynamo in einer Spule (Empfängerstromkreis) eine elektrische Spannung und kann damit nachgewiesen werden. Ihre Amplitude ist proportional zur transversalen Magnetisierung.

Nach Abschalten des hochfrequenten Wechselfeldes nimmt die transversale Magnetisierung (wieder) ab, die Spins richten sich also wieder parallel zum statischen Magnetfeld aus. Für diese sogenannte Relaxation benötigen sie eine charakteristische Abklingzeit. Diese ist von der chemischen Verbindung und der molekularen Umgebung abhängig, in der sich der präzedierende Wasserstoffkern befindet. Daher unterscheiden sich die verschiedenen Gewebearten charakteristisch in ihrem Signal, was zu verschiedenen Signalstärken (Helligkeiten) im resultierenden Bild führt.

Eine relativ junge Entwicklung ist die Kombination von PET und MRI in einem gemeinsamen Gerät als neue diagnostische Methode für den klinischen Einsatz. Sie ermöglichen kombinierte PET/MRI-Untersuchungen. Dabei stehen im Wesentlichen zwei Ansätze zur Verfügung.

Aus der US-Patentanmeldung US 2017 / 164 915 A1 sowie der US-Patentanmeldung US 2016 / 103 194 sind PET/MRI Anordnungen bekannt.

Aus dem Artikel BO J PENG ET AL, "Studies of the interactions of an MRI system with the shielding in a combined PET/MRI scanner", PHYSICS IN MEDICINE AND BIOLOGY, Bristol GB, (20091211), vol. 55, no. 1, doi:10.1088/0031-9155/55/1/016, ISSN 0031-9155, Seiten 265 - 280 ist eine Anordnung mit gleicher Achsenausrichtung für ein kombiniertes PET7MRI Gerät bekannt.

In einem integrierten Ansatz wird der PET Detektor hinter einer Schirmung angebracht, sodass die HF-Spulen des MRI-Scanners im Wesentlichen den inneren Teil darstellen. Allerdings ist dieser Aufbau sehr komplex und daher kostenträchtig. Zudem verlangt dieser Aufbau eine große Fläche und kann daher nicht überall realisiert werden. Bei simultan messenden PET-MRI-Systemen können die normalerweise in PET-Scannern eingesetzten Photomultiplier aufgrund des für die MRI-Bildgebung nötigen Magnetfelds nicht verwendet werden.

In einem getrennten Ansatz wird z.B. nur die Patientenliege geteilt und die Untersuchung mittels der Scanner wird in zeitlicher Abfolge in Bezug auf das zu untersuchende Areal durchgeführt. Typischerweise beträgt der Abstand zwischen den einzelnen Scannern 1 m und mehr. Häufig werden die an sich zeitlich und räumlich getrennten Untersuchungsergebnisse anschließend durch Software wieder zusammengeführt. Diese bisher bekannten Geräte haben eine gemeinsame Achse.

Metall am oder im Körper kann Nebenwirkungen und Bildstörungen im MRI verursachen. D.h. auch Metalle, die z.B. Bestandteil eines PET-Scanners sind, können stören. Umgekehrt können natürlich auch Teile des PET-Scanners durch induzierte Ströme beschädigt werden.

Ein weiteres Problem ist, dass für bestimmte Tumoren, eine Positionierung näher am potentiellen Tumor zu einer besseren Auflösung in Bezug auf den Ort führen könnte. Wird jedoch ein PET-Scanner analog zu einem MRI-Scanner mit einer großen Öffnung aufgebaut, sodass ein Patient durch die Öffnung hindurch geschoben werden könnte, so leidet die Ortsauflösung und/oder die Sensitivtät, da der von PET-Detektoren abdeckbare Raumwinkel mit zunehmender Entfernung geringer wird.

Zwar wäre es möglich, neue halbleiterbasierte Sensoren für die PET-Detektoren zu verwenden und damit eine Anordnung innerhalb eines MRI-Scanners zu ermöglichen, jedoch muss dann die PET-Detektor-Elektronik elektromagnetisch geschirmt werden, denn in der Anregungsphase werden durch einen MRI-Scanner teils sehr starke Wechselfelder erzeugt, die zu einer Störung der Elektronik, insbesondere von Verstärkern, bis hin zur Zerstörung führen kann. Zudem ist der MRI-Scanner in der Abklingphase - der eigentlichen Scanphase - sehr empfindlich in Bezug auf elektromagnetische Störungen, die von der Elektronik des PET-Scanners ausgehen kann.

Ausgehend von dieser Situation ist es Aufgabe der Erfindung einen PET-Detektor für einen kombinierten PET/MRI-Scanner zur Verfügung zu stellen, der es ermöglicht, einen oder mehrere Nachteile aus dem Stand der Technik zu vermeiden.

Die Aufgabe wird gelöst durch einen PET-Detektor für einen kombinierten PET/MRI-Scanner. Der PET-Detektor weist eine Schirmung gegen elektromagnetische Felder auf, sodass elektromagnetische Felder, welche durch den MRI-Scanner erzeugt werden, von dem PET-Detektor ferngehalten werden, und sodass elektromagnetische Felder von der Verarbeitungselektronik des PET-Detektors von einer Empfangsspule des MRI-Scanners ferngehalten wird. Der PET-Detektor ist dabei zur Verwendung innerhalb des MRI-Scanners geeignet, wobei die Scanrichtung des PET-Detektors im Wesentlichen nicht parallel zur Hauptachse des MRI-Scanners ausgerichtet ist. Die Schirmung des PET-PET-Detektors weist mindestens einen Schlitz auf, der zumindest in einer Projektion senkrecht zur Hauptachse des MRI-Scanners ist, um induzierte Ströme zu vermeiden.

In einer Ausführungsform der Erfindung weist die Schirmung ein faserverstärktes Material auf.

Gemäß einer weiteren Ausführungsform der Erfindung weist die Schirmung ein elektrisch leitfähiges Polymer auf.

In noch einer Ausführungsform der Erfindung weist die Schirmung Ruß auf.

Gemäß noch einer weiteren Ausführungsform der Erfindung weist die Schirmung mindestens zwei Schlitze senkrecht zur Hauptachse des MRI-Scanners auf, um induzierte Ströme zu vermeiden.

In einer weiteren Ausführungsform der Erfindung ist der PET-Detektor in Bezug auf den mindestens einen Schlitz öffenbar.

Gemäß einer weiteren Ausführungsform der Erfindung ist mindestens ein Schlitz sagittal, parasagittal oder transvers in Bezug auf einen zu untersuchenden Patienten angeordnet.

Gemäß einer Ausgestaltung der Erfindung kann ein erfindungsgemäßer PET-Detektor zur Bildgebung einer weiblichen Brust oder eines Skrotums verwendet werden.

Weitere vorteilhafte Ausgestaltungen sind insbesondere Gegenstand der abhängigen Ansprüche, der Beschreibung und der Figuren.

Nachfolgend wird die Erfindung näher unter Bezug auf die Figuren erläutert. In diesen zeigt:
- Fig. 1: eine beispielhafte Anordnung eines PET-Scanners gemäß Ausführungsformen der Erfindung in Zusammenhang mit einem MRI-Scanner,
- Fig. 2: ein Detail einer Ausführungsform eines erfindungsgemäßen PET-Scanners,
- Fig. 3: ein Detail einer weiteren Ausführungsform eines erfindungsgemäßen PET-Scanners,
- Fig. 4: ein weiteres Detail einer Ausführungsform eines erfindungsgemäßen PET-Scanners,
- Fig. 5: ein weiteres Detail einer weiteren Ausführungsform eines erfindungsgemäßen PET-Scanners,
- Fig. 6: noch ein weiteres Detail einer Ausführungsform eines erfindungsgemäßen PET-Scanners in einem ersten Zustand in einer perspektivischen Darstellung,
- Fig. 7: das Detail aus Fig. 6 eines erfindungsgemäßen PET-Scanners in einem zweiten Zustand in einer Aufsicht, und
- Fig. 8: ein Detail zur Verdeutlichung des Begriffes PET-Scanner.

### Detaillierte Beschreibung

Nachfolgend wird die Erfindung eingehender unter Bezugnahme auf die Figur dargestellt werden. Dabei ist anzumerken, dass unterschiedliche Aspekte beschreiben werden, die jeweils einzeln oder in Kombination zum Einsatz kommen können. D.h. jeglicher Aspekt kann mit unterschiedlichen Ausführungsformen der Erfindung verwendet werden, soweit nicht explizit als reine Alternative dargestellt.

Weiterhin wird nachfolgend der Einfachheit halber in aller Regel immer nur auf eine Entität Bezug genommen werden. Soweit nicht explizit vermerkt, kann die Erfindung aber auch jeweils mehrere der betroffenen Entitäten aufweisen. Insofern ist die Verwendung der Wörter "ein", "eine" und "eines" nur als Hinweis darauf zu verstehen, dass in einer einfachen Ausführungsform zumindest eine Entität verwendet wird.

Teile eines erfindungsgemäßen PET-Detektors 2 für einen kombinierten PET/MRI-Scanner 1 sind in den Figuren dargestellt.

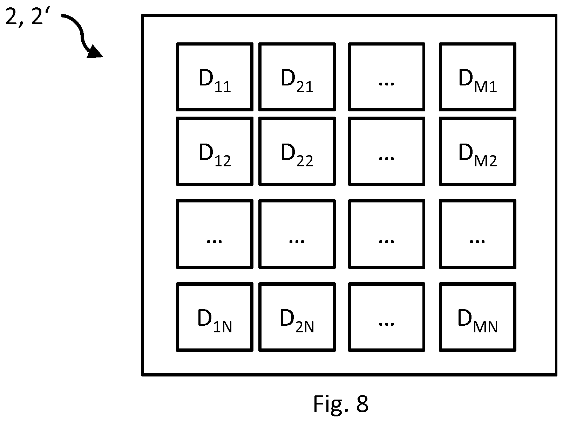

Der PET-Detektor 2 weist eine Vielzahl von einzelnen Detektoren D₁₁, ... D_{NM} auf, die um eine Achse B angeordnet sind. Ein solches Feld von einzelnen Detektoren D₁₁, ... D_{NM} ist in Figur 8 "abgewickelt" dargestellt.

Die Detektoren D₁₁, ... D_{NM} sind bevorzugt auf Halbleiterbasis, z.B. basierend auf Silizium (gegebenenfalls mit (Lawineneffekt-) Verstärkung), hergestellt. Solche Halbleiterdetektoren erlauben ein schlankeres Design, sodass die Detektoren in höherer Dichte und/oder auf kleinerem Raum zur Verfügung gestellt werden können. Hierdurch kann die Leistungsfähigkeit, insbesondere die räumliche Auflösung, stark erhöht werden. Insbesondere sind Halbleiterdetektoren aber auch innerhalb von MRI-Scannern einsetzbar.

Eine zugehörige Elektronik zur Auswertung kann unmittelbar an jedem einzelnen Detektor angeordnet oder aber in Gruppen organisiert sein. Dabei wird in üblicher Weise durch Erkennen von zwei Photonen durch gegenüberliegende Detektoren eine Emission durch den PET-Scanner 2 erkannt.

Der PET-Detektor 2 weist zudem eine Schirmung ES gegen elektromagnetische Felder auf, sodass elektromagnetische Felder, welche durch den MRI-Scanner 3 erzeugt werden, von dem PET-Detektor 2, insbesondere jedoch von der empfindlichen Verarbeitungs-/Verstärker-/Auswertelogik des PET-Detektors, ferngehalten wird. Die Schirmung ES hat zudem die Aufgabe elektromagnetische Felder von der Verarbeitungselektronik des PET-Detektors 2 von einer Empfangsspule des MRI-Scanners 3 fernzuhalten D.h. die Schirmung schützt zum einen den PET-Detektor 2, zum anderen auch den MRI-Scanner 3.

Der PET-Detektor 2 ist zur Verwendung innerhalb des MRI-Scanners 3 geeignet. Dabei ist die Scanrichtung/Achse B des PET-Detektors 2 im Wesentlichen nicht parallel zur Hauptachse A des MRI-Scanners 3 ausgerichtet. Die Hauptachse A des MRI-Scanners 3 ergibt sich im Wesentlichen als die Achse der Sende-Spule TX des MRI-Scanners 3 bzw. der Empfangsspule RX des MRI-Scanners 3.

Um zu vermeiden, dass Wirbelströme in der Anordnung des PET-Detektors 2 auftreten, die den PET-Detektor 2 stören könnten, die zudem zu einer starken Erwärmung und damit Verbrennungen am Patienten führen könnten, und die auch geeignet wären, das Messergebnis des MRI-Scanners 3 zu beeinträchtigen, weist die Schirmung ES des PET-Detektors 2 mindestens einen Schlitz S₁; S₂ auf, der zumindest in einer Projektion senkrecht zur Hauptachse des MRI-Scanners A ist, um induzierte Ströme zu vermeiden.

Auch wenn vorstehend der PET-Detektor 2 in Bezug auf eine Integration mit einem MRI-Scanner 3 beschrieben wurde, ist der PET-Detektor 2 nicht hierauf beschränkt und kann auch mit anderen Einrichtungen zur diagnostischen Bildgebung kombiniert werden.

Durch die vorstehende Anordnung ist es möglich, zum einen einen PET-Scan eines zu untersuchenden Gebietes als auch ein MRI-Scan eines zu untersuchenden Gebietes in hoher Auflösung bereit zu stellen.

Z.B. kann der PET-Detektor 2 mit einem MRI-Scanner 3 in einem gemeinsamen PET/MRI-Scan 1 eingebaut sein. Bevorzugt können z.B. für die Erkennung von Brustkrebs zwei PET-Detektoren 2, 2' unter einer Öffnung einer Patientenliege L vorgesehen sein. Die Patientin wird so auf der Patientenliege L positioniert, dass jede Brust in einen der PET-Detektoren 2, 2' eingeführt wird. So kann während eines MRI-Scans auch jede Brust durch einen PET-Detektoren 2, 2' gescannt werden. In dieser Anordnung ist es insbesondere auch möglich mittels des MRI-Scans Lymphknoten benachbart hierzu, z.B. axillare Lymphknoten, mit zu scannen. Bevorzugt können z.B. für die Erkennung von Hodenkrebs ein PET-Detektor 2 unter einer anderen Öffnung einer Patientenliege L vorgesehen sein (nicht dargestellt). Der Patient wird so auf der Patientenliege L positioniert, dass der Hoden in den PET-Detektor 2 eingeführt wird. So kann während eines MRI-Scans auch der Hoden durch einen PET-Detektor 2 gescannt werden. In dieser Anordnung ist es insbesondere auch möglich mittels des MRI-Scans Lymphknoten benachbart hierzu, z.B. retroperitoneale Lymphknoten, mit zu scannen.

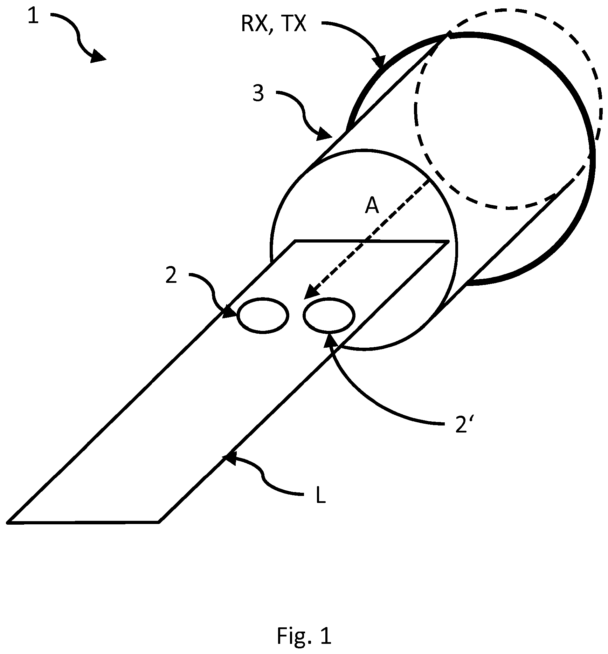

D.h. der PET-Detektor 2 bzw. die PET-Detektoren 2, 2' können geeignet im engen Raum unter der Patientenliege L angeordnet sein. Dabei ist z.B. für Untersuchungen der Brust zu bemerken, dass der Raum im Bereich des Brustbeins (sternum) gering ist, sodass die PET-Detektoren 2, 2' bei einer nebeneinanderliegenden Anordnung bestimmte Größen nicht überschreiten dürfen Dies ist mit herkömmlichen PET-Detektoren nicht möglich. Hingegen erlaubt die Erfindung die nebeneinanderliegende Anordnung, wie in Figur 1 angedeutet.

Es sei angemerkt, dass man zwar im Prinzip überlegen könnte, einen kombinierten PET/MRI-Scanner nur für ein bestimmtes Gewebe bereitzustellen. Jedoch wäre es auf Grund der räumlichen Limitierungen kaum möglich, dies vernünftig zu integrieren. Zudem wäre es dann zwar möglich, das primär interessante Untersuchungsgebiet zu erfassen, jedoch würden z.B. Informationen in Bezug auf benachbarte Lymphknoten nicht mehr erfasst werden, wodurch der diagnostische Nutzen stark eingeschränkt wäre.

Mittels der Erfindung wird nun aber ein für den MRI-Scanner 3 transparenter PET-Detektor 2, 2' zur Verfügung gestellt.

Im Prinzip wäre es möglich, die PET-Detektor 2, 2' z.B. aus dünnen, länglichen und galvanisch getrennten Detektoren D₁₁, ... D_{NM} aufzubauen. Dann könnte das vom MRI-Scanner 3 bereitgestellte hochfrequente Wechselfeld durch die Lücken hindurch. Allerdings würde dies dazu führen, dass das Design der PET-Detektoren 2, 2' sehr aufwändig würde, da dann jeder einzelne Detektor D₁₁, ... D_{NM} eine eigene Energieversorgung, Datenverarbeitung, Datenweiterleitung erfordern würde. Weiterhin würde eine aufwändige Synchronisation benötigt werden, um eine sinnvolle Auswertung zu ermöglichen. Dies alles würde dazu führen, dass die Raumanforderungen steigen, sodass eine integrierte Anordnung nahezu unmöglich wird.

In der Anordnung der Figur 1, in der die Sendespule TX und die Empfangsspule RX aufgezeigt sind (während z.B. ein statischer Magnet und eine Gradientenfeldspule nicht dargestellt sind), kann man ein rotierendes Magnetfeld in zwei lineare Feldanteile zerlegen, wobei einer der Feldanteile F_{B} in Richtung der Achse B des PET-Detektor 2, 2' koaxial orientiert ist und einen Feldanteil F_{A}, der senkrecht hierzu ist, z.B. in Hauptscanrichtung A. In diesem Fall wäre der senkrechte Feldanteil zumindest teilweise durch den PET-Detektor 2, 2' in Abhängigkeit von der Schirmung ES abgeschirmt. Der koaxiale Feldanteil F_{B} jedoch würde einen Wirbelstrom in der ringförmigen abgeschirmten Anordnung erzeugen, wodurch gegenwirkende Felder erzeugt werden, die der entsprechenden Feldkomponente entgegenwirken, sodass der Feldanteil auf eine nicht verwertbare Feldstärke abfallen würde. Durch den Schlitz S₁; S₂ jedoch wird der Wirbelstromfluß in der ringartigen Gestaltung der PET-Detektoren 2, 2' unterbrochen. Damit wird der PET-Detektor 2, 2' aber zumindest für diesen Feldanteil (Semi-) transparent, sodass das Signal des MRI-Scanners 3 messtechnisch und diagnostisch sinnvoll verwertet werden kann. Es sei angemerkt, dass die Empfangsspule RX alternativ oder zusätzlich auch anders angeordnet sein kann, z.B. näher am zu untersuchenden Gebiet. Hierfür können z.B. Empfangsspulen in unmittelbarer Nachbarschaft des zu untersuchenden Gebiets innerhalb der Sendespule TX angeordnet sein.

In dieser Anordnung kann die Verarbeitungs-/Verstärker-/Auswertelogik des PET-Detektor 2, 2' in einer baumartigen Struktur organisiert sein.

In einer Ausführungsform der Erfindung weist die Schirmung ES eine faserverstärktes Material, beispielsweise ein Material mit Carbonfasern, Glasfasern, Kohlenstoffnanoröhren, auf. Weiterhin kann die Schirmung ES eine elektrisch leitfähiges Polymer aufweisen. Das elektrisch leitfähige Polymer kann dabei intrinsisch leitfähig sein, wie z.B. auf Basis von Polyanilin, und/oder durch Zugabe von Füll- bzw. Hilfsstoffen, wie z.B. Nickel oder Ruß, der Schirmung ES elektrische leitfähige Eigenschaften verleihen.

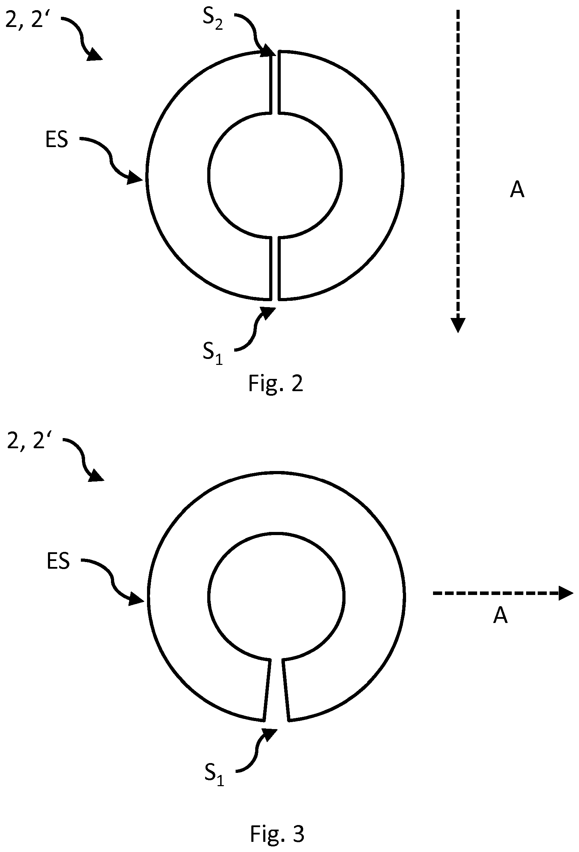

In einer Ausführungsform, die in Figur 2 dargestellt ist, ist aufgezeigt, dass die Schirmung ES des PET-Detektors auch mehr als einen Schlitz S1 aufweisen kann. Im Beispiel der Figur 2 weist die die Schirmung ES zwei Schlitze S₁; S₂ senkrecht zur Hauptachse A des MRI-Scanners 3 auf, um induzierte Ströme zu vermeiden.

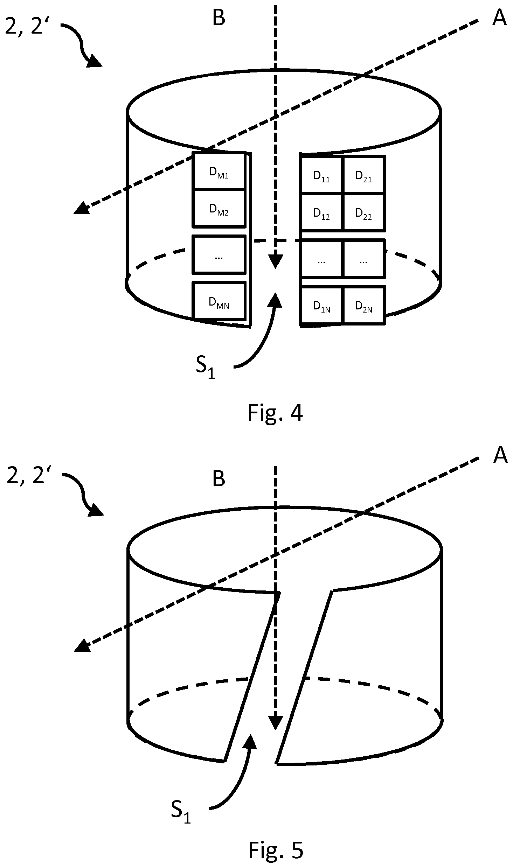

Es sei dabei angemerkt, dass die Art und Weise des Schlitzes S₁ / der Schlitze S₁, S₂ auch so gewählt sein kann, dass zumindest in einer Projektion des Schlitzes diese senkrecht zur Hauptachse A des MRI-Scanners 3 ist. D.h. auch andere Schlitzformen, wie z.B. in Figur 5 gezeigt, oder auch kompliziertere Schlitzprofile, wie z.B. treppenartige Gestaltungen, sind durch die Erfindung möglich und erlauben dennoch die Bereitstellung der elektrischen Eigenschaften.

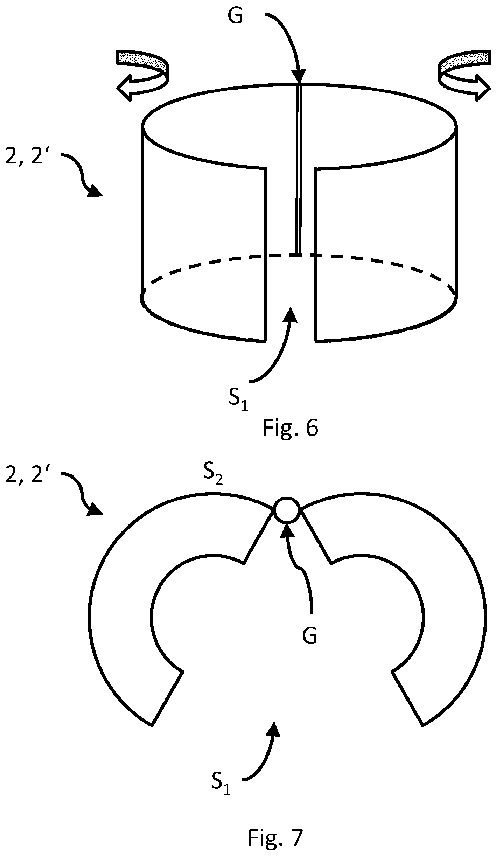

Zudem kann - wie in Figur 6 und 7 gezeigt - durch die Schlitze S₁, S₂ eine Öffnung des PET-Detektors 2, 2' ermöglicht werden, um z.B. ein zu untersuchendes Gewebe problemlos in den geöffneten PET-Detektor 2, 2' (Figur 7) einführen zu können. Anschließend kann der PET-Detektor 2, 2' "geschlossen werden", z.B. indem in Bezug auf ein Scharnier G, welches am Schlitz S2 angeordnet ist, die beiden Teilhälften des PET-Detektor 2, 2' relativ zueinander bewegt werden (Figur 6). Natürlich kann auch eine der Teilhälften des PET-Detektor 2, 2' feststehend sein, z.B. durch Anbringung an der Patientenliege L.

Dabei können der Schlitz / die Schlitze S₁; S₂ im Wesentlichen sagittal, parasagittal oder transverse in Bezug auf einen zu untersuchenden Patienten angeordnet sein.

## Patentansprüche

1. PET-Detektor (2) für einen kombinierten PET/MRI-Scanner (1), der PET-Detektor (2) aufweisend
• eine ringförmige Schirmung (ES) gegen elektromagnetische Felder, sodass elektromagnetische Felder, welche durch den MRI-Scanner (3) erzeugt werden, von dem PET-Detektoren (2) ferngehalten wird, und sodass elektromagnetische Felder von Verarbeitungselektronik des PET-Detektors (2) von einer Empfangsspule des MRI-Scanners (3) ferngehalten wird,
• wobei der PET-Detektor (2) zur Verwendung innerhalb des MRI-Scanners (3) geeignet ist,
• wobei die Scanrichtung (B) des PET-Detektors (2) im Betrieb in einem kombinierten PET/MRI-Scanner (1) im Wesentlichen senkrecht zur Hauptachse (A) des MRI-Scanners (3) ausgerichtet ist, wobei die Hauptachse A des MRI-Scanners (3) sich als die Achse einer Sende-Spule (TX) des MRI-Scanners (3) bzw. einer Empfangsspule (RX) des MRI-Scanners ergibt,
• wobei die ringförmige Schirmung (ES) des PET-Detektors (2) mindestens einen Schlitz (S₁; S₂) bildet, der zumindest in einer Projektion senkrecht zur Hauptachse des MRI-Scanners (A) ist, um induzierte Ströme zu vermeiden,
• wobei die ringförmige Schirmung (ES) des PET-Detektors (2) bis auf den mindestens einen Schlitz (S₁; S₂) geschlossen ist.

2. PET-Detektor (2) nach Anspruch 1, **dadurch gekennzeichnet, dass** die Schirmung (ES) eine faserverstärktes Material aufweist.

3. PET-Detektor (2) nach Anspruch 1 oder 2, **dadurch gekennzeichnet, dass** die Schirmung (ES) eine elektrisch leitfähiges Polymer aufweist.

4. PET-Detektor (2) nach einem der vorhergehenden Ansprüche, **dadurch gekennzeichnet, dass** die Schirmung (ES) Ruß aufweist.

5. PET-Detektor (2) nach einem der vorhergehenden Ansprüche, **dadurch gekennzeichnet, dass** die Schirmung (ES) mindestens zwei Schlitze (S₁; S₂) senkrecht zur Hauptachse (A) des MRI-Scanners aufweist, um induzierte Ströme zu vermeiden.

6. PET-Detektor (2) nach einem der vorhergehenden Ansprüche, **dadurch gekennzeichnet, dass** der PET-Detektor (2) in Bezug auf den mindestens einen Schlitz (S₁; S₂) öffenbar ist.

7. Verwendung eines PET-Detektor nach einem der vorhergehenden Ansprüche zur Bildgebung in Bezug auf einen Pateienten, wobei der mindestens eine Schlitz (S₁; S₂) sagittal, parasagittal oder transverse in Bezug auf einen zu untersuchenden Patienten angeordnet ist.

8. Verwendung eines PET-Detektors (2) nach einem der vorhergehenden Ansprüche 1 bis 6 zur Bildgebung einer weiblichen Brust.

9. Verwendung eines PET-Detektors (2) nach einem der vorhergehenden Ansprüche 1 bis 6 zur Bildgebung eines Skrotums.

## Claims

1. A PET-detector (2) for a combined PET/MRI scanner (1), the PET-detector (2) comprising
• an annular shield (ES) against electromagnetic fields, so that electromagnetic fields, which are generated by the MR-scanner (3), are kept away from the PET-detectors (2), and so that electromagnetic fields of processing electronics of the PET-detector (2) are kept away from a receiving coil of the MRI-scanner (3),
• wherein the PET-detector (2) is suitable for use inside the MRI-scanner (3),
• wherein the scanning direction (B) of the PET-detector (2) in operation in a combined PET/MRI-scanner (1) is aligned essentially perpendicular to the main axis (A) of the MRI-scanner (3), wherein the main access A of the MRI-Scanner (3) emerges as the axis of a transmitting coil (TX) of the MRI-scanner (3) or a receiving coil (RX) of the MRI-scanner,
• wherein the annular shield (ES) of the PET-detector (2) forms at least one slot (S1; S2), which at least in one projection is perpendicular to the main axis of the MRI-scanner (A) in order to prevent induced currents,
• wherein the annular shield (ES) of the PET-detector (2) is closed except for the at least one slot (S1; S2).

2. The PET-detector (2) according to claim 1, **characterised in that** the shield (ES) comprises a fibre-reinforced material.

3. The PET-detector (2) according to claim 1 or 2, **characterised in that** the shield (ES) comprises an electrically conductive polymer.

4. The PET-detector (2) according to any one of the preceding claims, **characterised in that** the shield (ES) comprises soot.

5. The PET-detector (2) according to any one of the preceding claims, **characterised in that** the shield (ES) comprises at least two slots (S1; S2) perpendicular to the main axis (A) of the MRI-scanner, in order to prevent induced currents.

6. The PET-detector (2) according to any one of the preceding claims, **characterised in that** the PET-detector (2) can be opened with respect to the at least one slot (S1; S2).

7. Use of a PET-detector according to any one of the preceding claims for imaging in respect of a patient, wherein the at least one slot (S1, S2) is arranged sagittal, parasagittal or transverse with respect to a patient to be examined.

8. The use of a PET-detector (2) according to any one of preceding claims 1 to 6 for imaging a female breast.

9. The use of a PET-detector (2) according to any one of preceding claims 1 to 6 for imaging a scrotum.

## Revendications

1. Détecteur TEP (2) pour un appareil d'analyse combiné TEPIRM (1), le détecteur TEP (2) comportant
• un blindage annulaire (ES) contre les champs électromagnétiques de telle manière que les champs électromagnétiques, lesquels sont produits par l'appareil d'analyse IRM (3), sont maintenus éloignés du détecteur TEP (2), et de telle manière que les champs électromagnétiques du système électronique de traitement du détecteur TEP (2) sont maintenus éloignés par une bobine de réception de l'appareil d'analyse IRM (3),
• sachant que le détecteur TEP (2) est adapté à l'utilisation à l'intérieur de l'appareil d'analyse IRM (3),
• sachant que la direction de balayage d'analyse (B) du détecteur TEP (2) est orientée en fonctionnement dans un appareil d'analyse TEP/IRM combiné (1) pour l'essentiel perpendiculairement à l'axe principal (A) de l'appareil d'analyse IRM (3), sachant que l'axe principal A de l'appareil d'analyse IRM (3) se présente comme l'axe d'une bobine d'émission (TX) de l'appareil d'analyse IRM (3) ou d'une bobine de réception (RX) de l'appareil d'analyse IRM,
• sachant que le blindage annulaire (ES) du détecteur TEP (2) forme au moins une fente (S1 ; S2), qui est au moins dans une projection perpendiculaire à l'axe principal de l'appareil d'analyse IRM (A) pour éviter des flux induits,
• sachant que le blindage annulaire (ES) du détecteur TEP (2) est fermé jusque sur au moins une fente (S1 ; S2).

2. Détecteur TEP (2) selon la revendication 1, **caractérisé en ce que** le blindage (ES) comporte un matériau renforcé aux fibres.

3. Détecteur TEP (2) selon la revendication 1 ou 2, **caractérisé en ce que** le blindage (ES) comporte un polymère électroconducteur.

4. Détecteur TEP (2) selon l'une quelconque des revendications précédentes, **caractérisé en ce que** le blindage (ES) comporte de la suie.

5. Détecteur TEP (2) selon l'une quelconque des revendications précédentes, **caractérisé en ce que** le blindage (ES) comporte au moins deux fentes (S1 ; S2) perpendiculairement à l'axe principal (A) de l'appareil d'analyse IRM pour éviter des flux induits.

6. Détecteur TEP (2) selon l'une quelconque des revendications précédentes, **caractérisé en ce que** le détecteur TEP (2) peut être ouvert par rapport à au moins une fente (S1 ; S2).

7. Utilisation d'un détecteur TEP selon l'une quelconque des revendications précédentes pour l'imagerie en rapport avec un patient, sachant qu'au moins une fente (S1 ; S2) est disposée de façon sagittale, parasagittale ou transversale par rapport à un patient à examiner.

8. Utilisation d'un détecteur TEP (2) selon l'une quelconque des revendications précédentes 1 à 6 pour l'imagerie d'une poitrine féminine.

9. Utilisation d'un détecteur TEP (2) selon l'une quelconque des revendications précédentes 1 à 6 pour l'imagerie d'un scrotum.
